# FASCICULE DE BREVET EUROPEEN

(11) **EP 0 465 293 B1**
(45) Date de publication et mention de la délivrance du brevet: **13.09.1995**
(21) Numéro de dépôt: 91401684.5
(22) Date de dépôt: 21.06.1991
(51) Int. Cl.: C07C 69/732, C07C 69/738, C07C 67/343, C07D 309/22, C07D 333/24

(54) **Nouveaux acrylates à fonction alcool, aldéhyde et/ou éther, leur procédé de fabrication et leur application à l'obtention de nouveaux polymères et copolymères**
Eine Alkohol-, Aldehyd- und/oder Ätherfunktion enthaltende Acrylate, ihr Herstellungsverfahren und ihre Anwendung bei Polymeren und Copolymeren
Acrylates having an alcohol, aldehyde and/or ether function, their process of preparation and their use for obtaining polymers and copolymers

(30) Priorité: 27.06.1990 FR 9008108
(43) Date de publication de la demande: 08.01.1992
(73) Titulaire: ELF ATOCHEM S.A., 92800 Puteaux (FR)
(72) Inventeur: Berthe, Marie-Christine, F-54500 Vandoeuvre les Nancy (FR); Caubere, Paul, F-54500 Nancy (FR); Fort, Yves, F-54500 Vandoeuvre les Nancy (FR)

(56) Documents cités:
- EP-A- 0 196 708
- TETRAHEDRON LETTERS, vol. 30, no. 46, 1989, pages 6337-6338, Pergamon Press plc, Oxford, GB; P. BAUCHAT et al.: "Synthesis of diaza [7,7] paracyclophanetetraene and diaza [7,2,7,2] paracyclophanehexaene"
- TETRAHEDRON LETTERS, vol. 44, no. 15, 1988, pages 4653-4670, Pergamon Press plc, GB; S.E. DREWES et al.: "Synthetic potential of the tertiary-amine- catalysed reaction of activated vinyl carbanions with aldehydes"
- TETRAHEDRON, vol. 48, No 31, 1992, pages 6371-6384.

## Description

La présente invention se rapporte à de nouveaux acrylates porteurs d'au moins deux fonctions choisies parmi les fonctions alcool, aldéhyde et éther, à un procédé pour leur fabrication et à l'obtention de nouveaux polymères et copolymères à partir desdits acrylates.

On connaît par le brevet US-A-3 743 669 un procédé de préparation de composés acryliques de formule 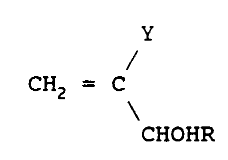
par réaction en phase liquide à une température de 0° à 200°C environ d'un dérivé d'acide carboxylique α,β-oléfiniquement insaturé de formule CH₂ = CHY avec un aldéhyde de formule RCHO, Y étant choisi parmi 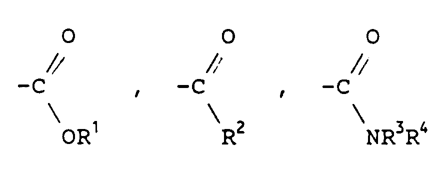
et -C≡N,
chacun de R¹ à R⁴ représentant un alkyle (C₁-C₁₂), cycloalkyle (C₅-C₁₂), aryle, aralkyle ou alkaryle, et R représentant alkyle (C₁-C₈), alk-(C₁-C₄)aryle, aralkyle (C₁-C₄) ou aryle, ladite réaction étant effectuée en présence d'une quantité catalytique d'une amine tertiaire cyclique possédant au moins un atome d'azote commun à trois cycles. Cette réaction est très lente à température ambiante. Ainsi la réaction de 7,9 moles d'acétaldéhyde et 5,27 moles d'acrylate d'éthyle à température ambiante en présence de 0,26 mole de diazabicyclo-[2,2,2]-octane produit-elle 93% de (hydroxy-1 éthyl)-2-acrylate d'éthyle après 7 jours seulement. La même réaction effectuée à 120-124°C procède avec une conversion de 82% au bout de 8 heures mais au détriment de la sélectivité. Par ailleurs la demanderesse a trouvé que cette réaction ne se produit pas, même au bout de 10 jours, avec certains aldéhydes dont le groupement carbonyle est encombré stériquement tel que le triméthylacétaldéhyde.

Compte tenu du comportement particulier que l'on peut attendre d'acrylates contenant un groupe hydroxy dans leurs structure, la demanderesse s'est intéressée à l'obtention de nouveaux acrylates par fonctionnalisation au moyen de dialdéhydes. A ce propos, on connaît déjà, par Tetrahedron letters, 30(46) 6337-6338 (1989) la préparation du 3-(4-carboxaldéhydephényl)-3-hydroxy-2-méthylène propanoate de méthyle (page 6337, composé 4) et du 1,4-di(3-hydroxy-2-méthylène carboxylate de méthyle)-benzène (composé 2) par reaction d'acrylate de méthyle avec du téréphtalaldéhyde et du diazabicyclo[2.2.2]octane.

Un premier objet de la présente invention consiste donc en de nouveaux acrylates choisis parmi ceux de formule 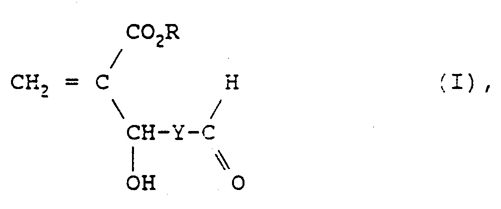
ceux de formule 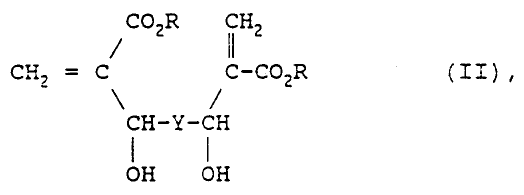
ceux de formule 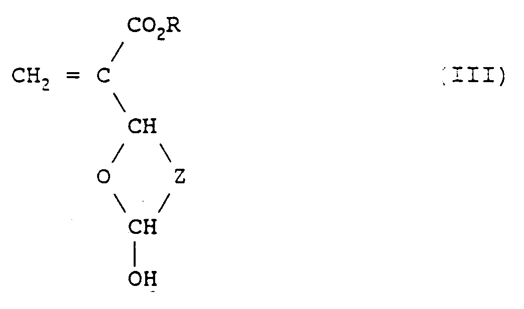
dans lesquelles :
- R est un radical choisi parmi les radicaux alkyles ayant de 1 à 12 atomes de carbone, les radicaux cycloalkyles ayant de 5 à 12 atomes de carbone, les radicaux aryles, arylalkyles et alkylaryles,
- Y est un radical choisi parmi les radicaux alkylène ayant de 1 à 12 atomes de carbone, les radicaux arylène ayant de 6 à 12 atomes de carbone, les radicaux hétérocycliques dont le cycle comporte de 5 à 10 chaînons et dont l'hétéroatome est choisi parmi l'azote, l'oxygène et le soufre, et les radicaux alkylarylène dont la partie alkyle comprend de 1 à 4 atomes de carbone, et Y ne pouvant représenter phénylène-1,4 lorsque R représente méthyle
- Z est un radical hydrocarboné comprenant au moins deux atomes de carbone formant avec l'oxygène et les deux carbones adjacents un cycle comportant de 4 à 8 chaînons, et de préférence comportant 5 ou 6 chaînons.

Comme exemples de radicaux R on peut citer notamment les radicaux méthyle, éthyle, n-propyle, isopropyle, n-butyle, isobutyle, phényle et benzyle. Comme exemples de radicaux Y on peut citer les radicaux (CH₂)ₙ dans lesquels n est un nombre entier allant de 4 à 12, le radical phénylène C₆H₄, les radicaux 3,3'-diphényle et 4,4'-diphényle, le radical 2,5-thiophényle et le radical 2,5-furyle.

Comme exemples de radicaux Z on peut citer notamment les radicaux (CH₂)₂ et (CH₂)₃ et le radical phénylène C₆H₄.

Un second objet de la présente invention consiste en un procédé de préparation des acrylates de formules (I) à (III). Quoique tous ces composés aient en commun pour leur préparation une étape mettant en jeu un acrylate dérivé d'un alcool ROH et un dialdéhyde, leur synthèse présente toutefois des particularités selon qu'il s'agit de monoacrylates de formules (I) et (III) ou bien de diacrylates de formule (II). C'est pourquoi le procédé de préparation selon l'invention sera maintenant décrit par référence à chacune des familles de composés selon l'invention.

La préparation des acrylates de formule (I) à (III) s'effectue en faisant réagir un acrylate de formule : 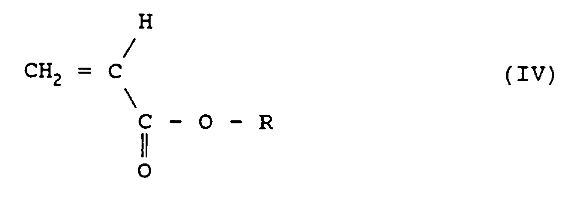
dans laquelle R est défini comme précédemment avec un dialdéhyde de formule 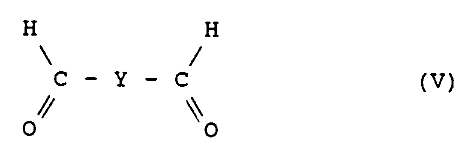
dans laquelle Y est défini comme précédemment, en présence d'une quantité efficace d'au moins un catalyseur de fonctionnalisation. Comme catalyseur de fonctionnalisation convenant bien à la réaction avec un aldéhyde on peut citer notamment des bases relativement fortes telles que des amines tertiaires cycliques ayant au moins un atome d'azote commun à trois cycles, décrites dans le brevet US-A-3 743 669, par exemple le diazabicyclo-[2,2,2]-octane, la quinuclidine, et l'α-quinuclidinol. Une quantité efficace de catalyseur de fonctionnalisation dépend évidemment de la nature de ce dernier, mais aussi de l'acrylate de formule (IV) et du dialdéhyde de formule (V). Elle est généralement comprise entre 2 et 20% environ, de préférence entre 4 et 10% environ en moles par rapport à la somme des réactants en présence - acrylate et dialdéhyde.

Comme exemple de dialdéhydes de formule (V) on peut citer notamment le téréphtalaldéhyde, l'isophtalaldéhyde, l'orthophtalaldéhyde, le glutaraldéhyde, l'hexanedial, le décanedial, le dodécanedial, le thiophène-2,5-dicarboxaldéhyde, le 3,3'-biformyldiphényl et le 4,4'-biformyldiphényl.

Le procédé de préparation selon l'invention peut être effectué en outre en présence d'au moins un activateur électrophile tel que le chlorure ou le fluorure de lithium dans une quantité pouvant atteindre jusqu'à 5% en moles environ par rapport à la somme des réactants en présence - acrylate et dialdéhyde.

Le procédé selon l'invention peut en outre être effectué en présence d'un solvant organique tel que le tétrahydrofuranne, le chlorure de méthylène, le chloroforme, le dioxanne, l'acétonitrile, la méthyléthylcétone, l'éthanol, l'acétate d'éthyle, etc.

Pour la mise en oeuvre du procédé selon l'invention, on peut choisir une température comprise entre 15°C et 125°C, de préférence entre 20°C et 50°C environ et ne dépassant pas la température d'ébullition du solvant lorsqu'un solvant est utilisé.

Les réactants peuvent être présents dans le milieu réactionnel en n'importe quelle concentration. Toutefois pour des raisons de cinétique, il est généralement préférable d'éviter une trop grande dilution par le solvant et de respecter un rapport molaire 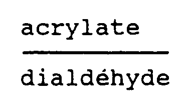
compris entre 0,2 et 10 environ. L'excès éventuel de l'un des deux réactants par rapport à l'autre lui permet alors de jouer le rôle de solvant.

Enfin, la réaction selon l'invention peut être effectuée en présence d'une quantité efficace d'au moins un inhibiteur de polymérisation. Comme exemples d'inhibiteurs de polymérisation utilisables, on peut citer notamment la phénothiazine, l'éther méthylique de l'hydroquinone, la N,N-diéthyl-hydroxylamine, le nitrobenzène, le di-tertiobutylcatéchol, l'hydroquinone, le p-anilinophénol, le phosphite de di-(2-éthylhexyl)-octylphényle, le 2,5-ditertiobutyl-4-hydroxytoluène, le bleu de méthylène et leur mélanges en toutes proportions. Une quantite efficace d'innibiteur de polymérisation est généralement comprise encre 0,05% et 0,5% en poids de composé acrylique.

Quoique la pression atmosphérique soit généralement satisfaisante, le procédé selon l'invention peut également être mis en oeuvre sous pression.

Selon que l'on désire orienter la réaction selon l'invention vers la production de monoacrylates de formules (I) et (III) ou bien vers la production de diacrylates de formule (II), il y a toutefois lieu de respecter certaines conditions particulières. En effet la réaction selon l'invention produit généralement un mélange d'un monoacrylate et d'un diacrylate qu'il convient ensuite, au moment choisi pour l'arrêt de la réaction, de séparer par une technique conventionnelle de séparation. Dans un certain nombre de cas, la séparation est facilitée par la différence d'état physique, à pression et température ordinaires, entre les deux composés formés. Ainsi par exemple dans le cas du téréphtalaldéhyde le monoacrylate formé est un liquide tandis que le diacrylate formé est un solide blanc. Comme on l'a déjà mentionné par ailleurs, les monoacrylates de formule (III) sont formés préférentiellement aux monoacrylates de formule (I) lorsque le dialdéhyde de formule (V) utilisé pour la réaction selon l'invention est de structure telle qu'il puisse facilement se produire une cyclisation intégrant l'un des atomes d'oxygène du dialdéhyde. Une telle possibilité est particulièrement favorable lorsque Y est un radical (CH₂)₂ ou (CH₂)₃ ou bien un radical phénylène C₆H₄ sur lequel les fonctions aldéhydes sont situées en position ortho.

La composition du mélange de monoacrylate et de diacrylate formé par la réaction selon l'invention est influencée de manière complexe par un ensemble de paramètres tels que :
- la durée de la réaction, celle-ci pouvant aller de 1 heure environ jusqu'à 12 jours environ, selon la température de réaction et la pression choisies ; de manière générale le monoacrylate est formé en premier lieu, de manière quasi-quantitative, au bout d'un temps relativement court, de l'ordre de quelques heures à température ambiante ; dans un second temps le monoacrylate disparaît progressivement au profit de la formation du diacrylate.
- la nature du solvant utilisé : toutes conditions étant égales par ailleurs, le diacrylate se forme beaucoup plus facilement dans le tétrahydrofuranne ou le chlorure de méthylène que dans le chloroforme.
- la quantité de solvant utilisé : la dilution par un solvant tel que le tétrahydrofuranne ralentit la première étape de formation du monoacrylate et, beaucoup plus encore la seconde étape de formation du diacrylate.
- la quantité de catalyseur de fonctionnalisation utilisé : toutes conditions étant égales par ailleurs, l'augmentation de la quantité de catalyseur conduit à une légère accélération de l'étape de formation de monoacrylate et à une accélération plus prononcée de l'étape de formation du diacrylate.
- le rapport molaire 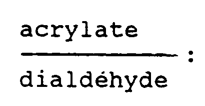 en présence de solvant et à volume total constant, c'est-à-dire hors effet de dilution, l'augmentation de ce rapport entraîne l'accélération de la formation du diacrylate.
- la température réactionnelle : toutes conditions étant égales par ailleurs, l'augmentation de la température se traduit par une formation plus rapide du diacrylate.

Les nouveaux acrylates selon l'invention peuvent se polymériser ou se copolymériser avec d'autres monomères à insaturation éthylénique, tels que l'éthylène, ainsi que :
- un acrylate ou méthacrylate d'alkyle dont le groupe alkyle linéaire ou ramifié, le cas échéant substitué, par exemple par au moins un atome d'halogène comme le chlore ou le fluor et/ou par au moins un groupe hydroxyle, possède de 1 à 20 atomes de carbone,
- un acrylate ou méthacrylate d'aryle tel que le méthacrylate de benzyle,
- un hydrocarbure vinylaromatique tel que le styrène, le vinyltoluène, l'alphaméthylstyrène, le méthyl-4 styrène, le méthyl-3 styrène, le méthoxy-4 styrène, l'hydroxyméthyl-2 styrène, l'éthyl-4 styrène, l'éthoxy-4 styrène, le diméthyl-3,4 styrène, le chloro-2 styrène, le chloro-3 styrène, le chloro-4 méthyl-3 styrène, le tert.-butyl-3 styrène, le dichloro-2,4 styrène, le dichloro-2,6 styrène et le vinyl-1 naphtalène,
- un nitrile insaturé tel que l'acrylonitrile ou le méthacrylonitrile,
- une maléimide N-substituée telle que la N-éthylmaléimide, la N-isopropylmaléimide, la N-n-butylmaléimide, la N-isobutylmaléimide, la N-terbutylmaléimide, la N-n-octylmaléimide, la N-cyclohexylmaléimide, la N-benzylmaléimide et la N-phénylmaléimide,
- un anhydride d'acide dicarboxylique insaturé tel que l'anhydride maléique, l'anhydride itaconique, l'anhydride citraconique ou l'anhydride tétrahydrophtalique,
- l'acide acrylique ou méthacrylique,
- un acrylate ou méthacrylate de polyol comme les diacrylates et diméthacrylates de l'éthylèneglycol, du propylèneglycol, du 1,3-butanediol, du 1,4-butanediol, du 1,6-hexane-diol, du néopentylglycol, du 1,4-cyclohexane-diol, du 1,4-cyclohexanediméthanol, du 2,2,4-triméthyl-1,3-pentanediol, du 2-éthyl-2-méthyl-1,3-propanediol, du 2,2-diéthyl-1,3-propanediol, du diéthylèneglycol, du dipropylèneglycol, du triéthylèneglycol, du tripropylèneglycol, du tétraéthylèneglycol, du tétrapropylèneglycol, du triméthyloléthane, du triméthylolpropane, du glycérol, du pentaérythritol, les triacrylates et triméthacrylates du triméthyloléthane, du triméthylolpropane, du glycérol, du pentaérythritol, les tétraacrylates et tétraméthacrylates du pentaérythritol, les di(méth)acrylates à hexa(méth)acrylates du dipentaérythritol, les poly(méth)acrylates de polyols mono- ou polyéthoxylés ou mono- ou polypropoxylés tels le triacrylate et le triméthacrylate du triméthylolpropane triéthoxylé, du triméthylolpropane tripropoxylé ; le triacrylate et le triméthacrylate du glycérol tripropoxylé ; le triacrylate, le triméthacrylate, le tétraacrylate et le tétraméthacrylate du pentaérythritol tétraéthoxylé,
- un acrylate ou méthacrylate époxydé choisi parmi le 2-époxyéthylbicyclo[2.2.1]hept-5(6)-yl (méth)acrylate, l'acrylate d'époxydicyclopentyloxyéthyle ainsi que ceux de formule : 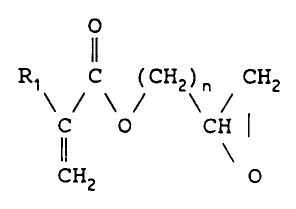 dans laquelle R₁ est choisi parmi l'atome d'hydrogène et le radical méthyle, et n est un nombre entier allant de l à 16, ceux de formule : 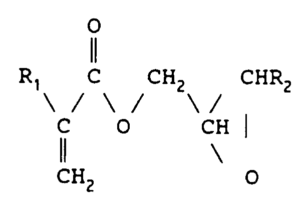 dans laquelle R₁ est choisi parmi l'atome d'hydrogène et le radical méthyle, et R₂ est choisi parmi les radicaux alkyle ayant de 1 à 12 atomes de carbone et les radicaux aryle ayant de 6 à 12 atomes de carbone,
   et ceux de formules : 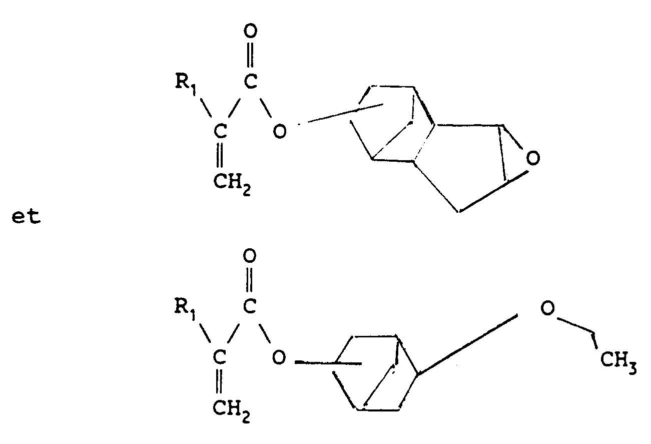 dans lesquelles R₁ est choisi parmi l'atome d'hydrogène et le radical méthyle,
- un acrylamide ou méthacrylamide, un acrylate ou méthacrylate de dialkylaminoalkyle et leurs sels quaternaires,
- l'acrylate et le méthacrylate de (norbornyloxy-2)-2 éthyle et de (diméthanodécahydronaphtyloxy-2)-2 éthyle, et
- des oxazolidones acryliques et méthacryliques choisies parmi celles de formule : 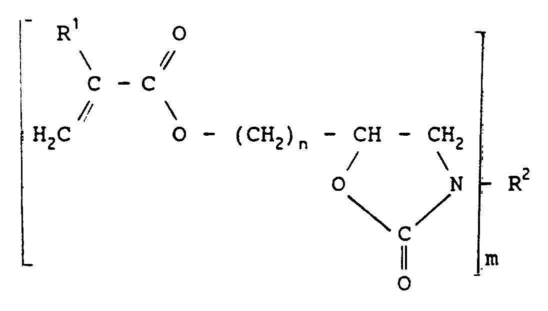 et celles de formule : 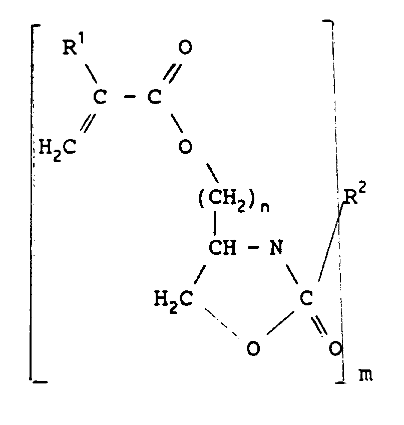 formules dans lesquelles :
   - R¹ est choisi parmi l'atome d'hydrogène et le radical méthyle,
   - n est un nombre entier allant de 1 à 12,
   - m est un nombre entier allant de 1 à 3, et
   - R² est un radical hydrocarboné, alkyle linéaire, ramifié ou cyclique ou bien aromatique, possédant de 5 à 12 atomes de carbone,
   lesdites oxazolidones pouvant être obtenues par réaction, entre 30°C et 90°C, d'un composé portant une fonction (méth)acrylique avec un composé portant au moins une fonction isocyanate,
- des composés acryliques et méthacryliques choisis parmi ceux de formule : 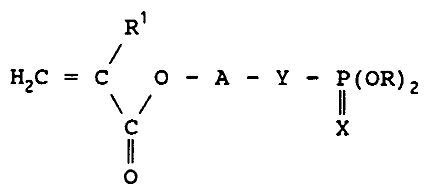 dans laquelle :
   - R¹ est choisi parmi l'atome d'hydrogène et le radical méthyle,
   - A est choisi parmi les radicaux (CH₂)ₙ pour lesquels n est un nombre entier de 2 à 12 et le radical -(CH₂CH₂O)_{d}-CH₂CH₂-, d étant un nombre entier allant de 1 à 20,
   - X est choisi parmi les atomes de soufre et d'oxygène,
   - Y est choisi parmi les atomes de soufre et d'oxygène,
   avec la condition que X est un atome de soufre et Y est un atome d'oxygène lorsque A est le radical -(CH₂CH₂O)_{d}-CH₂CH₂-, et
   - R est choisi parmi les radicaux alkyle ayant de 1 à 20 atomes de carbone et les groupes -(CH₂)ₚSR³ dans lesquels p est un nombre entier allant de 3 à 12 et R³ est un radical alkyle ayant de 1 à 20 atomes de carbone,
   ceux de formule : 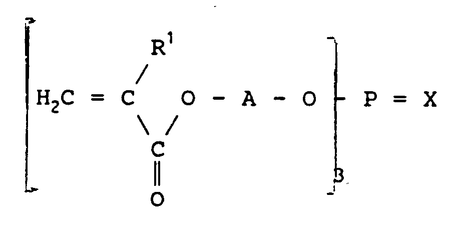 dans laquelle :
   - R¹ est choisi parmi l'atome d'hydrogène et le radical méthyle,
   - A est choisi parmi les radicaux (CH₂)ₙ pour lesquels n est un nombre entier de 2 à 12 et le radical -(CH₂CH₂O)_{d}-CH₂CH₂-, d étant un nombre entier allant de 1 à 20, et
   - X est choisi parmi les atomes de soufre et d'oxygène,
   et ceux de formule : 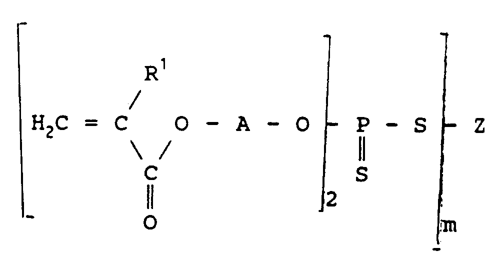 dans laquelle :
   - R¹ est choisi parmi l'atome d'hydrogène et le radical méthyle,
   - A est choisi parmi les radicaux (CH₂)ₙ pour lesquels n est un nombre entier de 2 à 12,
   - m est un nombre entier allant de 1 à 3, et
   - Z est choisi parmi l'atome d'hydrogène, les radicaux R²QH, R² étant un radical alkyle ayant de 2 à 12 atomes de carbone et Q étant choisi parmi les atomes d'oxygène et de soufre, et les atomes des métaux des groupes IA, IIA, IIIA, IB, IIB, VIB, VIIB et VIII de la Classification Périodique,
   avec la condition que Z est choisi parmi l'atome d'hydrogène et les radicaux R²OH lorsque m = 1 et que m est la valence de Z lorsque Z est un métal. De tels composés peuvent être préparés par réaction d'un composé acrylique ou méthacrylique de formule : 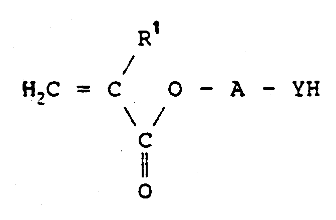 dans laquelle R¹, A et Y ont les mêmes significations que dans la formule (I), avec un composé pentavalent du phosphore, celui-ci pouvant être par exemple un composé de formule PXT₃ dans laquelle X a la même signification que dans la formule (II) et T désigne un atome d'halogène, ou bien un composé phosphoré de formule : 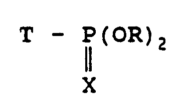 dans laquelle R et X ont les mêmes significations que dans la formule (I) et T désigne un atome d'halogène, ou bien le pentasulfure P₂S₅,
et de manière générale tous monomères à insaturation éthylénique capables de se copolymériser par un processus radicalaire sous l'effet d'un générateur de radicaux libres tel que micro-ondes, rayonnement bêta, gamma ou ultraviolet ou initiateur chimique tel que persulfate, peroxyde, hydroperoxyde ou composé diazoïque.

Ainsi un troisième objet de la présente invention consiste en un polymère comprenant dans sa chaîne au moins un motif dérivé d'un nouvel acrylate de formule (I), (II) ou (III) à la condition que Y puisse aussi représenter phénylène-1,4 lorsque R représente méthyle, et, le cas échéant, au moins un motif dérivé d'un autre monomère à insaturation éthylénique tel que décrit ci-dessus. Dans le cas des copolymères, les conditions de polymérisation seront choisies les plus proches possibles de celles habituellement employées pour la polymérisation du comonomère à insaturation éthylénique, à savoir par exemple :
- une température de 140°C à 300°C et une pression de 1000 à 3000 bars environ lorsque ce comonomère est l'éthylène,
- une température de 30°C à 90°C environ lorsque ce comonomère est un composé acrylique ou méthacrylique,
- une température de 80°C à 200°C environ lorsque ce comonomère est un hydrocarbure vinylaromatique.

Les exemples suivants sont donnés à titre illustratif et non limitatif de la présente invention.

### EXEMPLES 1 à 25

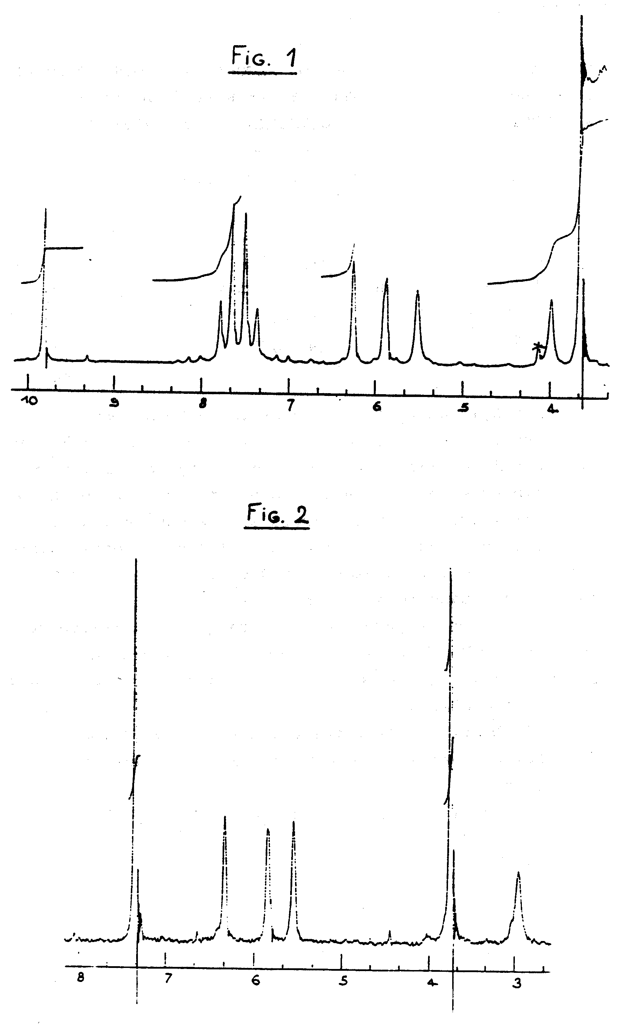

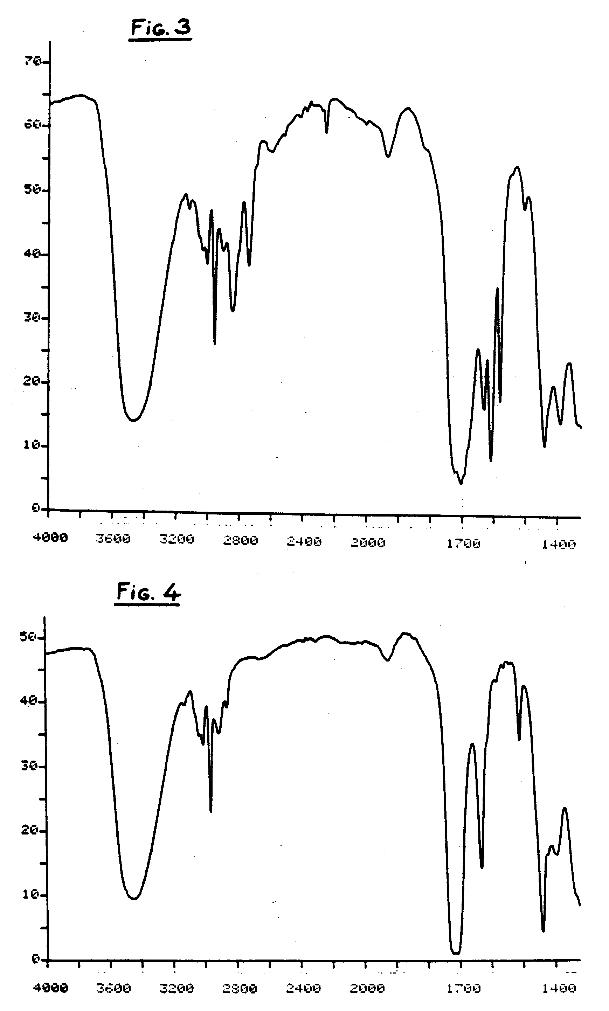

Dans un ballon en verre de volume 3 l muni d'un réfrigérant, on introduit successivement 26,8 g (0,2 mole) de téréphtalaldéhyde, x mole de diazabicyclo[2.2.2.]octane, y mole d'acrylate de méthyle et z ml de solvant. Le mélange est agité à la température T (exprimée en degrés Celsius) pendant la durée t (exprimée en heures). On ajoute alors au mélange 200 ml de chlorure de méthyle et 120 ml d'acide chlorhydrique 1N. La phase aqueuse est extraite une fois par 40 ml de chlorure de méthylène. La phase organique est lavée deux fois par 80 ml d'eau, puis séchée sur du sulfate de magnésium. Après évaporation du solvant, le mono- ou diacrylate formé est purifié sur une colonne de silice, l'éluant étant un mélange comprenant 35% d'acétate d'éthyle et 65% d'éther de pétrole. Les pourcentages M de monoacrylate et D de diacrylate présents dans le mélange réactionnel à l'issue du temps t sont évalués par chromatographie en phase gazeuse et reportés dans le tableau I ci-après en même temps que les paramètres réactionnels. Le monoacrylate (3-(4-carboxaldéhydephényl)-3-hydroxy-2-méthylène propanoate de méthyle) est un liquide incolore. Le diacrylate (1,4-di(3-hydroxy-2-méthylène carboxylate de méthyle)-benzène) est un solide blanc. Tous deux ont été identifiés par les techniques suivantes :
- résonance magnétique nucléaire du proton, utilisant un spectromètre JEOL PMX 60SI : les spectres obtenus comportent des déplacements chimiques à :
   a) 9,8 (s,1H) 7,3-7,8 (m,4H) 6,2 (m,1H) 5,9 (m,1H) 5,5 (m,1H) 3,9 (m,1H,OH) 3,6 (s,3H) en ppm pour le monoacrylate (Figure 1).
   b) 7,3 (s,4H) 6,3 (m,2H) 5,8 (m,2H) 5,5 (m,2H) 3,7 (s,6H) 2,9 (m,2H,OH) en ppm pour le diacrylate (Figure 2).
- spectrophotométrie infrarouge, utilisant un spectromètre PERKIN ELMER 841 : les spectres obtenus comportent des bandes caractéristiques à :
   c) cm⁻¹ 3477, 1724, 1703, 1631, 1609, 1579, 1439 pour le monoacrylate (Figure 3).
   d) cm⁻¹ 3459, 1724, 1631 et 1439 pour le diacrylate (Figure 4).

**TABLEAU I**

| Exemple | x | y | z | solvant | T | t | M | D |
|---|---|---|---|---|---|---|---|---|
| 1 | 0,1 | 1,0 | 50 | THF | 20 | 8 | 98 | 2 |
| 2 | 0,1 | 1,0 | 50 | THF | 20 | 96 | 46 | 54 |
| 3 | 0,1 | 1,0 | 50 | THF | 20 | 192 | 35 | 65 |
| 4 | 0,1 | 1,0 | 50 | THF | 20 | 288 | 19 | 81 |
| 5 | 0,1 | 1,0 | 50 | CH₂Cl₂ | 20 | 8 | 95 | 5 |
| 6 | 0,1 | 1,0 | 50 | CH₂Cl₂ | 20 | 96 | 35 | 65 |
| 7 | 0,1 | 1,0 | 50 | CH₂Cl₂ | 20 | 192 | 26 | 74 |
| 8 | 0,1 | 1,0 | 50 | CH₂Cl₂ | 20 | 240 | 23 | 77 |
| 9 | 0,1 | 1,0 | 50 | CHCl₃ | 20 | 96 | 97 | 3 |
| 10 | 0,1 | 0,4 | 0 | --- | 20 | 2,5 | 96 | 4 |
| 11 | 0,1 | 0,4 | 10 | THF | 20 | 3 | 94 | 2 |
| 12 | 0,1 | 0,4 | 44 | THF | 20 | 3 | 94 | 3 |
| 13 | 0,1 | 0,4 | 44 | THF | 20 | 24 | 82 | 18 |
| 14 | 0,1 | 0,4 | 44 | THF | 20 | 96 | 50 | 50 |
| 15 | 0,1 | 0,4 | 164 | THF | 20 | 96 | 98 | 2 |
| 16 | 0,4 | 2,0 | 20 | THF | 20 | 96 | 30 | 70 |
| 17 | 0,4 | 2,0 | 20 | THF | 20 | 240 | 9 | 91 |
| 18 | 0,1 | 0,2 | 50 | THF | 20 | 8 | 80 | 0 |
| 19 | 0,1 | 0,2 | 50 | THF | 20 | 24 | 96 | 2 |
| 20 | 0,1 | 1,0 | 0 | --- | 20 | 2 | 85 | 4 |
| 21 | 0,4 | 2,0 | 20 | THF | 45 | 24 | 53 | 47 |
| 22 | 0,4 | 2,0 | 20 | THF | 45 | 72 | 19 | 81 |
| 23 | 0,4 | 2,0 | 20 | THF | 45 | 192 | 4 | 96 |
| 24 | 0,4 | 2,0 | 20 | THF | 55 | 24 | 61 | 39 |
| 25 | 0,4 | 2,0 | 20 | THF | 55 | 72 | 18 | 82 |
| 26* | 0,1 | 1,0 | 0 | --- | 20 | 0,8 | 95 | 5 |

| | | | | | | | | |
|---|---|---|---|---|---|---|---|---|
| * essai réalisé en présence de chlorure de lithium. | | | | | | | | |
| THF = tétrahydrofuranne. | | | | | | | | |

### EXEMPLE 26

On reproduit la procédure expérimentale de l'exemple 20 aux deux exceptions suivantes près :
- 0,8 g (0,02 mole) de chlorure de lithium est ajouté au mélange réactionnel,
- la réaction est interrompue après 50 minutes.

L'analyse du mélange à l'issue de la réaction permet de déterminer les pourcentages M de monoacrylate et D de diacrylate indiqués au tableau.

### EXEMPLES 27 à 35

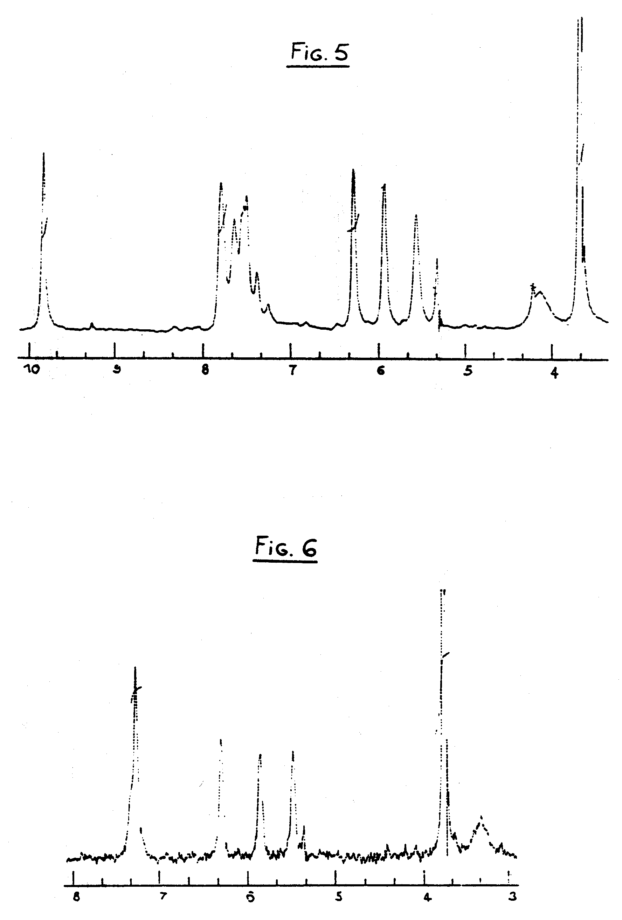

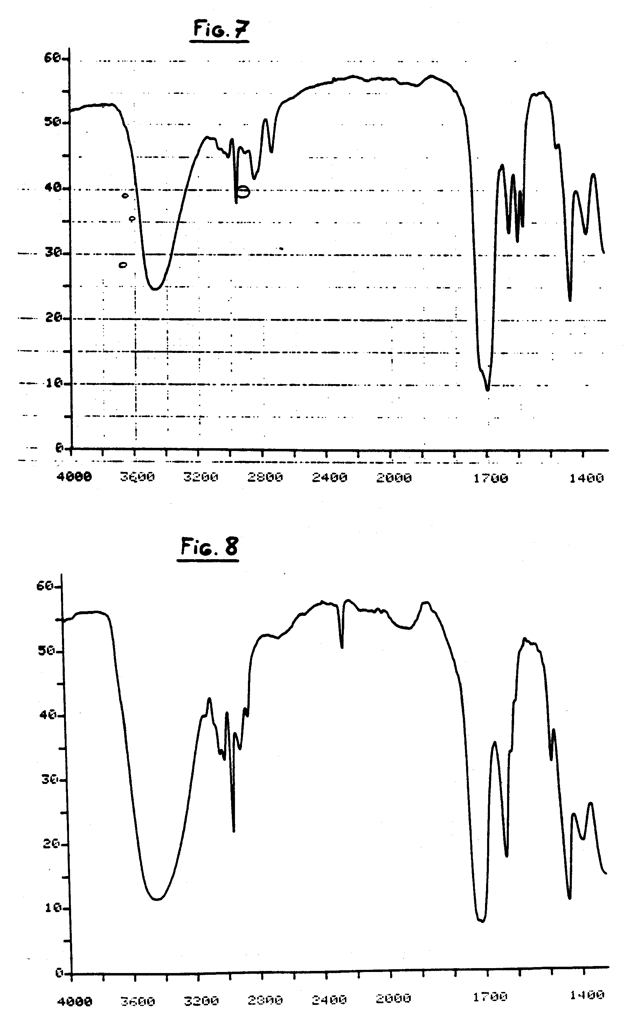

Dans un ballon en verre de volume 3 l, on introduit successivement 26,8 g (0,2 mole) d'isophtalaldéhyde, x mole de diazabicyclo[2.2.2]octane, y mole d'acrylate de méthyle, z ml de solvant et, le cas échéant, q mole de chlorure de lithium. Le mélange est agité à la température de 25°C pendant la durée t (exprimée en heures). Le mélange est ensuite traité comme aux exemples 1 à 25. Les pourcentages M de monoacrylate et D de diacrylate présents dans le mélange réactionnel à l'issue du temps t sont évalués par chromatographie en phase gazeuse et reportés dans le tableau II ci-après en même temps que les paramètres réactionnels. Le monoacrylate (3-(3-carboxaldéhydephényl)-3-hydroxy-2-méthylène propanoate de méthyle) et le diacrylate (1,3-di(3-hydroxy-2-méthylène carboxylate de méthyle)-benzène) sont des liquides incolores. Tous deux ont été identifiés par les mêmes techniques que celles mentionnées aux exemples 1 à 25 et présentent les caractéristiques spectrales suivantes :
- résonance magnétique nucléaire du proton :
   a) 9,7 (s,1H) 7,3-7,8 (m,4H) 6,2 (m,1H) 5,8 (m,1H) 5,5 (m,1H) 4,0 (m,1H,OH) 3,6 (s,3H) en ppm pour le monoacrylate (Figure 5).
   b) 7,3 (m,4H) 6,3 (m,2H) 5,8 (m,2H) 5,5 (m,2H) 3,7 (s,6H) 3,3 (m,2H,OH) en ppm pour le diacrylate (Figure 6).
- spectrophotométrie infrarouge :
   c) bandes d'absorption à 3471 cm⁻¹, 1723 cm⁻¹, 1701 cm⁻¹, 1630 cm⁻¹, 1603 cm⁻¹, 1587 cm⁻¹ et 1441 cm⁻¹ pour le monoacrylate (Figure 7).
   d) bandes d'absorption à 3458 cm⁻¹, 1723 cm⁻¹, 1632 cm⁻¹ et 1441 cm⁻¹ pour le diacrylate (Figure 8).

**TABLEAU II**

| Exemple | x | y | z | q | solvant | t | M | D |
|---|---|---|---|---|---|---|---|---|
| 27 | 0,1 | 1,0 | 0 | 0 | - | 3 | 68 | 1 |
| 28 | 0,1 | 1,0 | 0 | 0 | - | 5 | 85 | 10 |
| 29 | 0,1 | 1,0 | 0 | 0 | - | 24 | 30 | 70 |
| 30 | 0,1 | 1,0 | 0 | 0,02 | - | 2 | 82 | 18 |
| 31 | 0,1 | 1,0 | 0 | 0,02 | - | 24 | 14 | 86 |
| 32 | 0,1 | 1,0 | 0 | 0,02 | - | 48 | 8 | 92 |
| 33 | 0,2 | 0,4 | 50 | 0 | THF | 3 | 37 | 0 |
| 34 | 0,2 | 0,4 | 50 | 0 | THF | 12 | 86 | 3 |
| 35 | 0,2 | 0,4 | 50 | 0,02 | THF | 3 | 90 | 3 |

### EXEMPLE 36

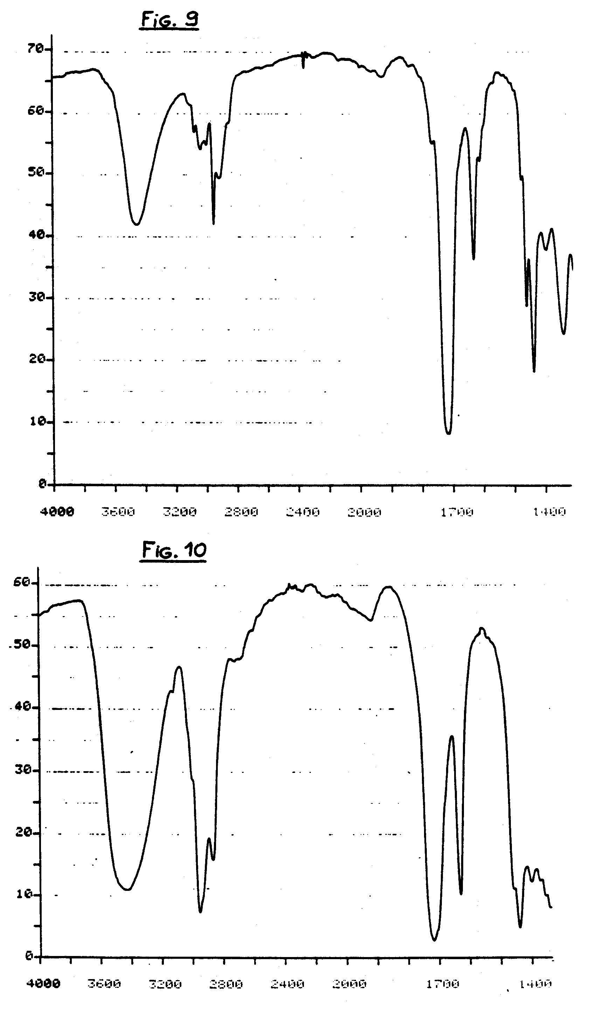

Dans un ballon en verre de volume 3 l, on introduit successivement 26,8 g (0,2 mole) d'orthophtalaldéhyde, 11,2 g (0,1 mole) de diazabicyclo[2.2.2]octane, 90 ml (1 mole) d'acrylate de méthyle et 50 ml de tétrahydrofuranne. Le mélange est agité à 250°C pendant 24 heures, puis traité comme aux exemples 1 à 25. L'analyse par chromatographie en phase gazeuse révèle la formation, avec un rendement de 86%, d'un composé identifié comme celui de formule 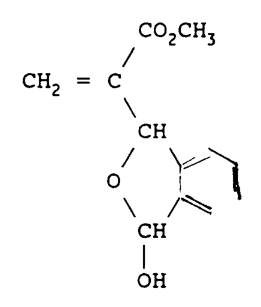
par spectrophotométrie infrarouge au moyen d'un spectromètre PERKIN-ELMER 841. Le spectre obtenu comporte des bandes caractéristiques à 3459 cm⁻¹, 1721 cm⁻¹, 1631 cm⁻¹, 1463 cm⁻¹ et 1439 cm⁻¹ (Figure 9).

### EXEMPLE 37

Dans un ballon en verre de volume 3 l, on introduit successivement 0,2 mode de glutaraldéhyde 50% aqueux, 1 mole d'acrylate de méthyle, 0,1 mole de diazabicyclo[2.2.2]octane, et 50 ml de dichlorométhane. Le mélange est agité à 25°C pendant 144 heures puis traité comme aux exemples 1 à 25. L'analyse par chromatographie en phase gazeuse révèle la formation, avec un rendement de 30%_{,} d'un composé identifié comme celui de formule 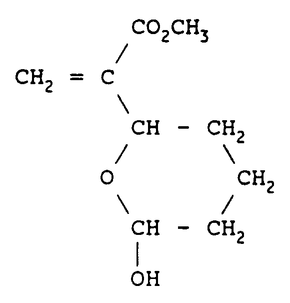
par spectrophotométrie infrarouge au moyen d'un spectromètre PERKIN-ELMER 841. Le spectre obtenu comporte des bandes caractéristiques à 3435, 1721 et 1632 cm⁻¹ (Figure 10).

### EXEMPLE 38

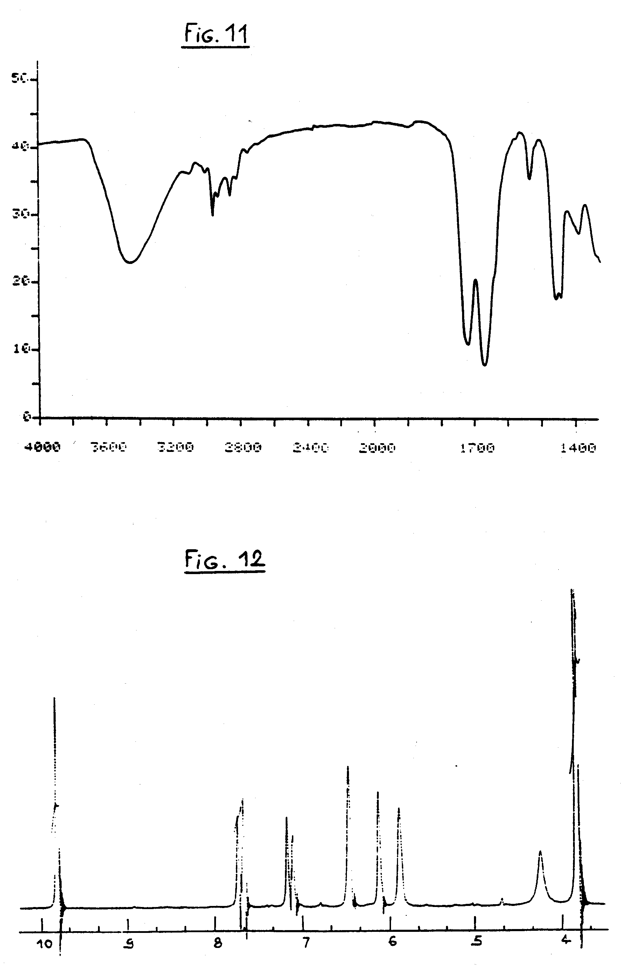

Dans un ballon en verre de volume 3 l, on introduit successivement 0,2 mole de thiophène-2,5-dicarboxaldéhyde, 0,4 mole d'acrylate de méthyle, 0,2 mole de diazabicyclo[2.2.2]octane et 50 ml de dichlorométhane. Le mélange est agité à 25°C pendant 105 minutes puis traité comme aux exemples 1 à 25. L'analyse par chromatographie en phase gazeuse révèle la formation, avec un rendement de 67%, d'un composé identifié comme celui de formule 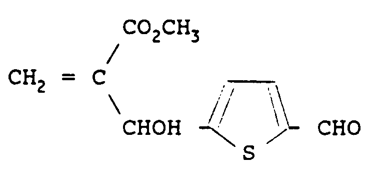
par les techniques suivantes :
- spectrophotométrie infrarouge : le spectre obtenu (Figure 11) révèle des bandes caractéristiques à 3462 cm⁻¹, 1718 cm⁻¹, 1670 cm⁻¹ et 1456 cm⁻¹.
- résonance magnétique nucléaire du carbone 13 : déplacements chimiques à 183,3 ppm, 165,9 ppm, 157,5 ppm, 142,3 ppm, 140,6 ppm, 136,9 ppm, 126,8 ppm, 125,6 ppm, 68,8 ppm et 52,0 ppm.
- résonance magnétique nucléaire du proton (Figure 12) : déplacements chimiques à 9,8 ppm (s,1H), 7,6 ppm (d,1H), 7,1 ppm (d,1H), 6,4 ppm (m,1H), 6,1 ppm (m,1H), 5,8 ppm (m,1H) 4,2 ppm (m,1H,OH) et 3,7 ppm (s,3H).

### EXEMPLE 39

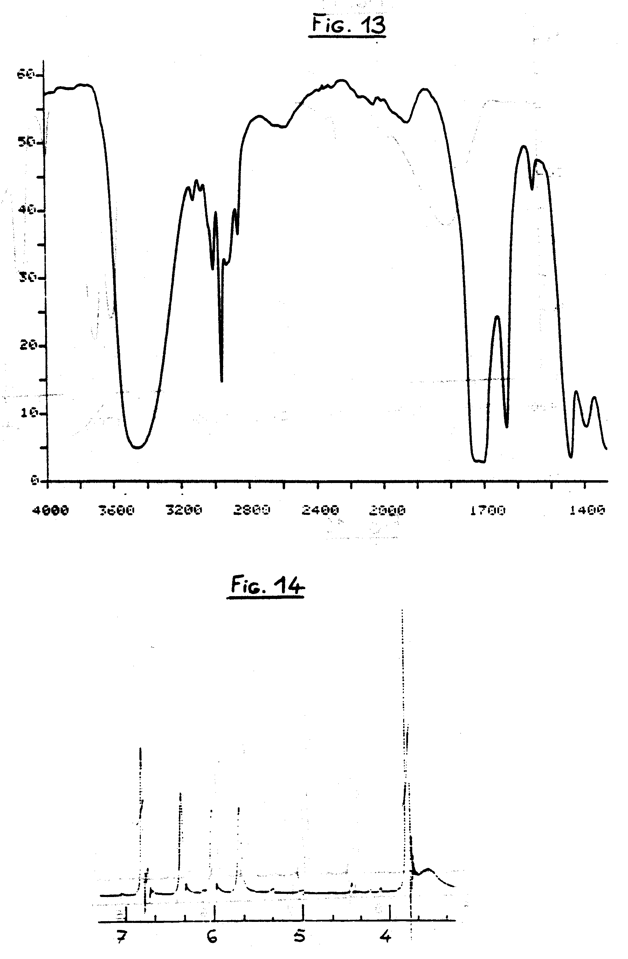

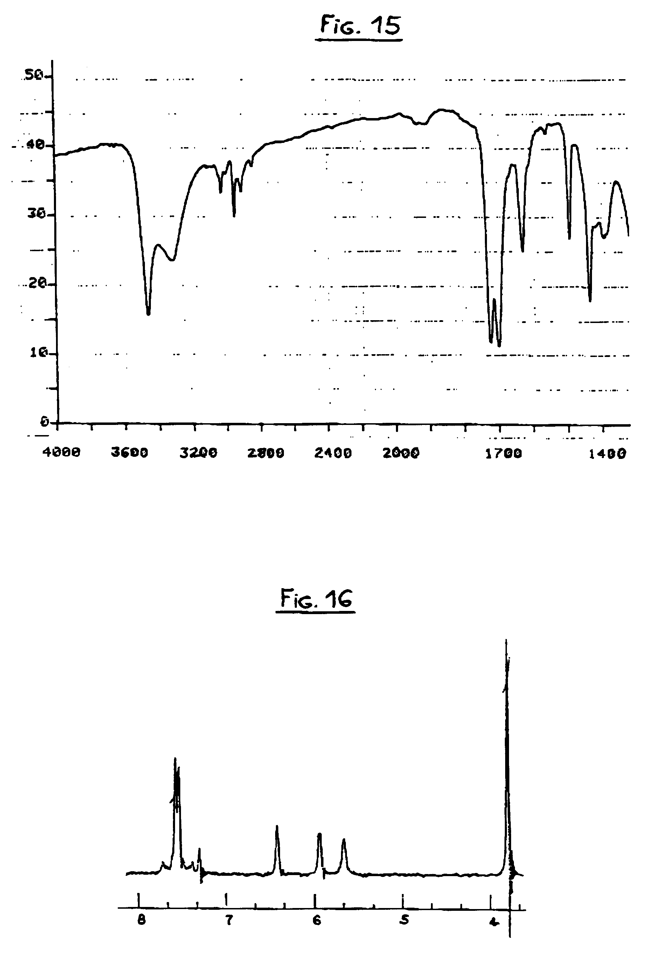

Dans un ballon en verre de volume 3 l, on introduit successivement 0,2 mole de thiophène-2,5-dicarboxaldéhyde, 2 moles d'acrylate de méthyle et 0,2 mole de diazabicyclo[2.2.2]octane. Le mélange est agité à 25°C pendant 46 heures puis traité comme aux exemples 1 à 25. L'analyse par chromatographie en phase gazeuse révèle la formation, avec un rendement de 35%_{,} du diacrylate de formule 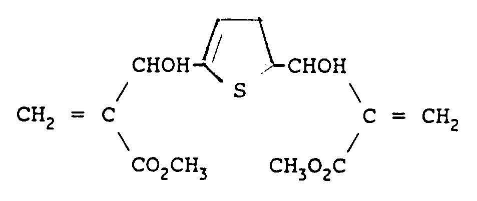
Celui-ci a été indentifié par :
- spectrophotométrie infrarouge : le spectre obtenu (Figure 13) révèle des bandes caractéristiques à 3460 cm⁻¹, 1709 cm⁻¹, 1632 cm⁻¹ et 1439 cm⁻¹.
- résonance magnétique nucléaire du proton (Figure 14) : déplacements chimiques à 6,7 ppm (s,2H), 6,3 ppm (m,2H), 6,0 ppm (m,2H), 5,7 ppm (m,2H), 3,7 ppm (s,6H) et 3,5 ppm (m,2H,OH).

### EXEMPLE 40

Dans un ballon en verre de volume 3 l, on introduit successivement 0,2 mole de 4,4'-biformyldiphényl, 1 mole d'acrylate de méthyle et 0,4 mole de diazabicyclo[2.2.2]octane. Le mélange est agité à 25°C pendant 168 heures puis traité comme aux exemples 1 à 25. L'analyse par chromatographie en phase gazeuse révèle la formation, avec un rendement de 60%, du diacrylate de formule 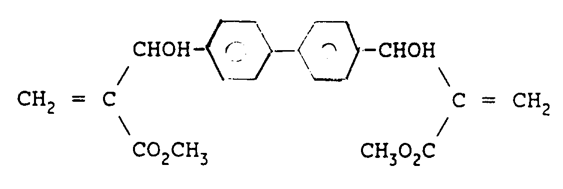
Celui-ci a été indentifié par :
- spectrophotométrie infrarouge : le spectre obtenu (Figure 15) révèle des bandes caractéristiques à 3466 cm⁻¹, 1724 cm⁻¹, 1702 cm⁻¹, 1629 cm⁻¹, 1494 cm⁻¹ et 1437 cm⁻¹.
- résonance magnétique nucléaire du proton (Figure 16) : déplacements chimiques à 7,6 ppm (m,8H), 6,4 ppm (m,2H), 5,9 ppm (m,2H), 5,6 ppm (m,2H) et 3,8 ppm (s,6H).

## Revendications

1. Acrylates choisis parmi ceux de formule 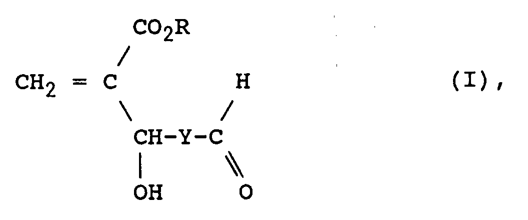 ceux de formule 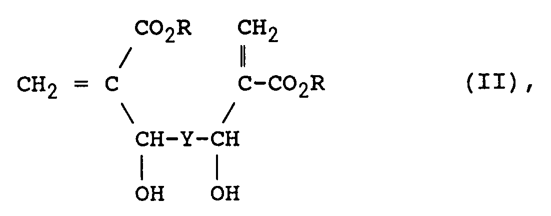 et ceux de formule 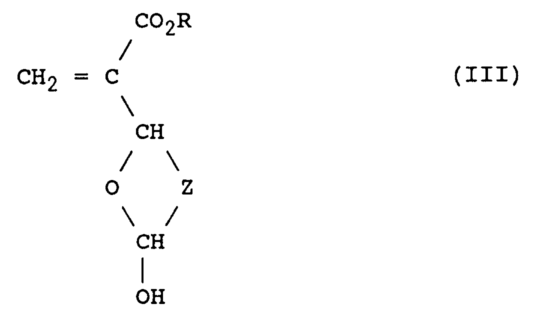 dans lesquelles :
- R est un radical choisi parmi les radicaux alkyles ayant de 1 à 12 atomes de carbone, les radicaux cycloalkyles ayant de 5 à 12 atomes de carbone, les radicaux aryles, arylalkyles et alkylaryles,
- Y est un radical choisi parmi les radicaux alkylène ayant de 1 à 12 atomes de carbone, les radicaux arylène ayant de 6 à 12 atomes de carbone, les radicaux hétérocycliques dont le cycle comporte de 5 à 10 chaînons et dont l'hétéroatome est choisi parmi l'azote, l'oxygène et le soufre, et les radicaux alkylarylène dont la partie alkyle comprend de 1 à 4 atomes de carbone, et Y ne pouvant représenter phénylène-1,4 lorsque R représente méthyle,
- Z est un radical hydrocarboné comprenant au moins deux atomes de carbone formant avec l'oxygène et les deux carbones adjacents un cycle comportant de 4 à 8 chaînons.

2. Acrylates selon la revendication 1, choisis parmi ceux de formules (I) et (II), caractérisés en ce que Y est choisi parmi les radicaux (CH₂)ₙ dans lesquels n est un nombre entier allant de 4 à 12, les radicaux phénylène C₆H₄, les radicaux diphényle-3,3' et diphényle-4,4', le radical 2,5-thiophényle et le radical 2,5-furyle.

3. Acrylates selon la revendication 1, choisis parmi ceux de formule (III), caractérisés en ce que Z est choisi parmi les radicaux (CH₂)₂ et (CH₂)₃ et le radical phénylène C₆H₄.

4. Procédé de préparation des acrylates selon la revendication 1, caractérisé en ce qu'on fait réagir un acrylate de formule : 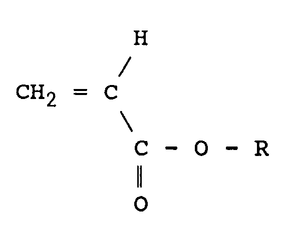 dans laquelle R est défini comme à la revendication 1, avec un dialdéhyde de formule 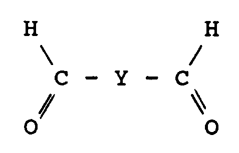 dans laquelle Y est défini comme à la revendication 1, en présence d'une quantité efficace d'au moins un catalyseur de fonctionnalisation consistant en au moins une amine tertiaire cyclique ayant au moins un atome d'azote commun à trois cycles.

5. Procédé selon la revendication 4, caractérisé en ce que le catalyseur de fonctionnalisation est utilisé à raison de 2 à 20% en moles par rapport à la somme de l'acrylate et du dialdéhyde.

6. Procédé selon l'une des revendications 4 et 5, caractérisé en ce qu'il est mis en oeuvre en présence d'au moins un activateur électrophile.

7. Procédé selon l'une des revendications 4 à 6, caractérisé en ce qu'il est mis en oeuvre en présence d'un solvant organique.

8. Procédé selon l'une des revendications 4 à 7, caractérisé en ce qu'il est mis en oeuvre à une température comprise entre 15°C et 125°C.

9. Procédé selon l'une des revendications 4 à 8, caractérisé en ce que le rapport molaire 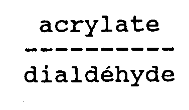 est compris entre 0,2 et 10.

10. Polymère comprenant dans sa chaîne au moins un motif dérivé d'un acrylate selon la revendication 1, à la condition que Y puisse aussi représenter phénylène-1,4 lorsque R représente méthyle.

## Claims

1. Acrylates chosen from those of formula 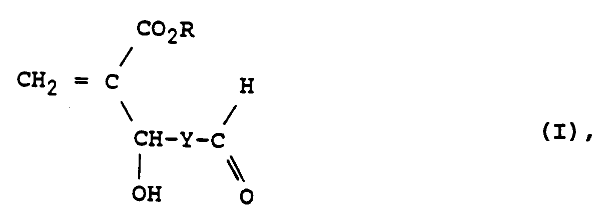 those of formula 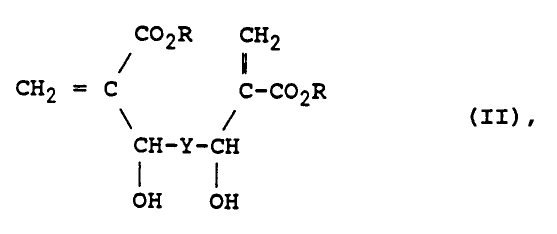 and those of formula 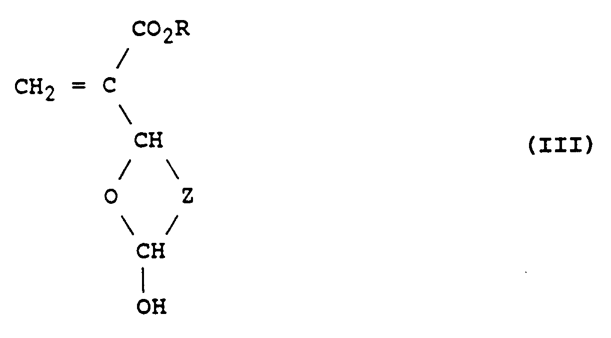 in which:
- R is a radical chosen from alkyl radicals containing from 1 to 12 carbon atoms, cycloalkyl radicals containing from 5 to 12 carbon atoms, and aryl, arylalkyl and alkylaryl radicals,
- Y is a radical chosen from alkylene radicals containing from 1 to 12 carbon atoms, arylene radicals containing from 6 to 12 carbon atoms, heterocyclic radicals whose ring contains from 5 to 10 members and whose heteroatom is chosen from nitrogen, oxygen and sulphur, and alkylarylene radicals in which the alkyl part contains from 1 to 4 carbon atoms, and Y not being able to denote 1,4-phenylene when R denotes methyl,
- Z is a hydrocarbon radical containing at least two carbon atoms forming with the oxygen and the two adjacent carbons a ring containing from 4 to 8 members.

2. Acrylates according to Claim 1, chosen from those of formulae (I) and (II), characterized in that Y is chosen from the radicals (CH₂)ₙ in which n is an integer ranging from 4 to 12, phenylene radicals C₆H₄, 3,3'-diphenyl and 4,4'-diphenyl radicals, the 2,5-thiophenyl radical and the 2,5-furyl radical.

3. Acrylates according to Claim 1, chosen from those of formula (III), characterized in that Z is chosen from the radicals (CH₂)₂ and (CH₂)₃ and the phenylene radical C₆H₄.

4. Process for the preparation of the acrylates according to Claim 1, characterized in that an acrylate of formula: 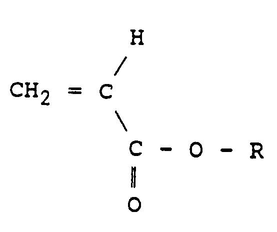 in which R is defined as in Claim 1, is reacted with a dialdehyde of formula 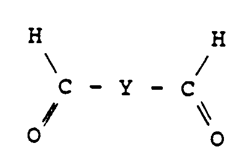 in which Y is defined as in Claim 1, in the presence of an effective quantity of at least one functionalization catalyst consisting of at least one cyclic tertiary amine containing at least one nitrogen atom common to three rings.

5. Process according to Claim 4, characterized in that the functionalization catalyst is employed in a proportion of 2 to 20 mol% relative to the sum of the acrylate and dialdehyde.

6. Process according to either of Claims 4 and 5, characterized in that it is carried out in the presence of at least one electrophilic activator.

7. Process according to one of Claims 4 to 6, characterized in that it is carried out in the presence of an organic solvent.

8. Process according to one of Claims 4 to 7, characterized in that it is carried out at a temperature of between 15°C and 125°C.

9. Process according to one of Claims 4 to 8, characterized in that the acrylate/dialdehyde molar ratio is between 0.2 and 10.

10. Polymer containing in its chain at least one unit derived from an acrylate according to Claim 1, on condition that Y may also denote 1,4-phenylene when R denotes methyl.

## Patentansprüche

1. Acrylate, die ausgewählt sind unter Acrylaten der Formeln 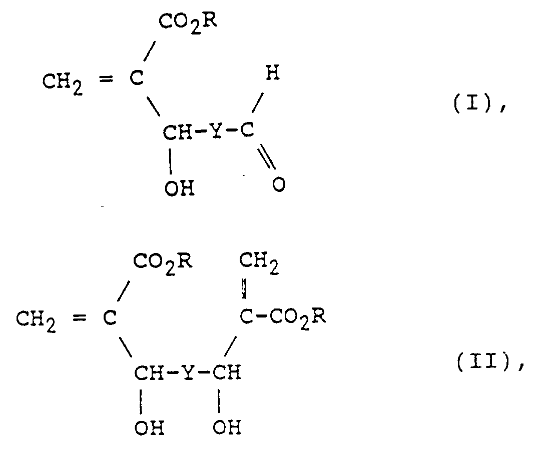 und 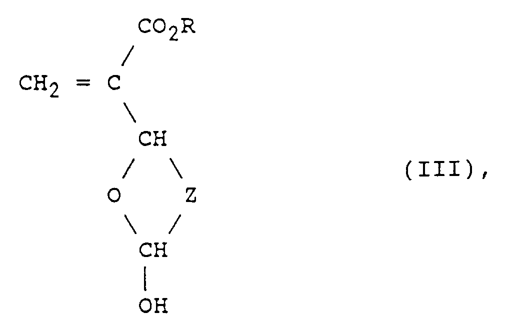 in denen bedeuten:
- R eine Gruppe, die ausgewählt ist unter Alkylgruppen mit 1 bis 12 Kohlenstoffatomen, Cycloalkylgruppen mit 5 bis 12 Kohlenstoffatomen sowie Aryl-, Arylalkyl- und Alkylarylgruppen,
- Y ein Gruppe, die ausgewählt ist unter Alkylengruppen mit 1 bis 12 Kohlenstoffatomen, Arylengruppen mit 6 bis 12 Kohlenstoffatomen, heterocyclischen Gruppen, deren Ring 5 bis 10 Ringglieder aufweist und deren Heteroatom unter Stickstoff, Sauerstoff und Schwefel ausgewählt ist, sowie Alkylarylengruppen, deren Alkylteil 1 bis 4 Kohlenstoffatome enthält, wobei Y kein 1,4-Phenylen sein kann, wenn R ein Methylrest ist,
- Z eine Kohlenwasserstoffgruppe mit mindestens zwei Kohlenstoffatomen, die mit dem Sauerstoff und den beiden benachbarten Kohlenstoffatomen einen Ring mit 4 bis 8 Ringgliedern bilden.

2. Acrylate nach Anspruch 1, die unter Acrylaten der Formeln (I) und (II) ausgewählt sind, dadurch gekennzeichnet, daß Y ausgewählt ist unter Gruppen (CH₂)ₙ, in denen n eine ganze Zahl von 4 bis 12 ist, Phenylengruppen C₆H₄, 3,3'-Biphenylylen und 4,4'-Biphenylylen, 2,5-Thienylen und 2,5-Furylen.

3. Acrylate nach Anspruch 1, die unter Acrylaten der Formel (III) ausgewählt sind, dadurch gekennzeichnet, daß Z unter (CH₂)₂ und (CH₂)₃ sowie Phenylen C₆H₄ ausgewählt ist.

4. Verfahren zur Herstellung der Acrylate nach Anspruch 1, dadurch gekennzeichnet, daß ein Acrylat der Formel 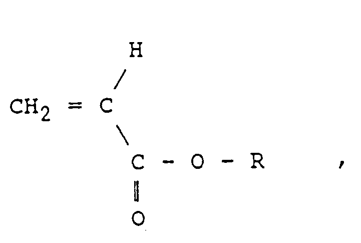 in der R wie in Anspruch 1 definiert ist, mit einem Dialdehyd der Formel 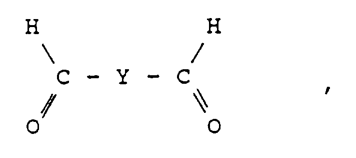 in der Y wie in Anspruch 1 definiert ist, in Anwesenheit einer wirksamen Menge von mindestens einem Funktionalisierungskatalysator, der aus mindestens einem cyclischen tertiären Amin mit mindestens einem drei Ringen angehörenden Stickstoffatom besteht, zur Reaktion gebracht wird.

5. Verfahren nach Anspruch 4, dadurch gekennzeichnet, daß der Funktionalisierungskatalysator in einem Verhältnis von 2 bis 20 Mol-%, bezogen auf die Summe an Acrylat und an Dialdehyd, eingesetzt wird.

6. Verfahren nach einem der Ansprüche 4 und 5, dadurch gekennzeichnet, daß es in Gegenwart von mindestens einem elektrophilen Aktivierungsreagens durchgeführt wird.

7. Verfahren nach einem der Ansprüche 4 bis 6, dadurch gekennzeichnet, daß es in Gegenwart eines organischen Lösungsmittels durchgeführt wird.

8. Verfahren nach einem der Ansprüche 4 bis 7, dadurch gekennzeichnet, daß es bei einer Temperatur von 15 bis 125 °C durchgeführt wird.

9. Verfahren nach einem der Ansprüche 4 bis 8, dadurch gekennzeichnet, daß das Molverhältnis Acrylat/Dialdehyd 0,2 bis 10 beträgt.

10. Polymer, das in seiner Kette mindestens eine von einem Acrylat nach Anspruch 1 abgeleitete Einheit enthält, mit der Maßgabe, daß Y auch 1,4-Phenylen sein kann, wenn R ein Methylrest ist.
